(19) Europäisches Patentamt
European Patent Office
Office européen des brevets

(11) Veröffentlichungsnummer: **0 047 950**
**B1**

(12) # EUROPÄISCHE PATENTSCHRIFT

(45) Veröffentlichungstag der Patentschrift: **28.09.83**

(51) Int. Cl.³: **C 07 C 141/04,** C 25 B 3/02

(21) Anmeldenummer: **81107002.8**

(22) Anmeldetag: **07.09.81**

(54) **Omega-Fluorsulfato-perfluorcarbonsäurederivate und Verfahren zu deren Herstellung.**

(30) Priorität: **12.09.80 DE 3034538**

(43) Veröffentlichungstag der Anmeldung: **24.03.82 Patentblatt 82/12**

(45) Bekanntmachung des Hinweises auf die Patenterteilung: **28.09.83 Patentblatt 83/39**

(84) Benannte Vertragsstaaten: **BE CH DE FR GB IT LI NL**

(56) Entgegenhaltungen: **DE-A-2 651 531**

(73) Patentinhaber: **HOECHST AKTIENGESELLSCHAFT, Postfach 80 03 20, D-6230 Frankfurt am Main 80 (DE)**

(72) Erfinder: **Millauer, Hans, Dr., An den sieben Bäumen 21b, D-6236 Eschborn (DE)**
Erfinder: **Schwertfeger, Werner, Dr., Erlenstrasse 8, D-6306 Langgöns (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 047 950 B1

BUNDESDRUCKEREI BERLIN

ω-Fluorsulfato-perfluorcarbonsäurederivate und Verfahren zu deren Herstellung

Perfluorierte organische Verbindungen sind Vor-, Zwischen- und Endprodukte auf verschiedenen Sachgebieten. Oligomere perfluorierte Vinylverbindungen sind z. B. wertvolle Schmieröle und Gleitsubstanzen mit außerordentlich hoher chemischer und thermischer Resistenz; höhermolekulare (polymere) derartige Verbindungen besitzen Bedeutung als chemisch und thermisch stabile Überzugs- und Dichtungsmaterialien, Elastomere, wenn noch basische oder saure Gruppen vorhanden sind: als Ionenaustauscher z. B. für Elektrolyse-Zellmembranen, etc.

Perfluorierte monomere Vinylverbindungen für derartige Einsatzzwecke sind beispielsweise bekannt aus der US-PS 3 282 875. Die darin beschriebenen Verbindungen besitzen die Formel:

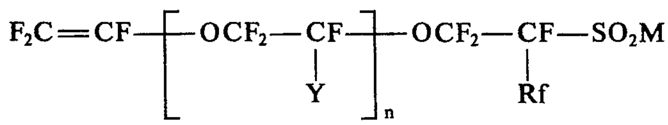

worin Rf = F oder ($C_1-C_{10}$)-Perfluoralkyl
Y = F oder $CF_3$
n = ganze Zahl von 1 – 3 und
M = F, OH, Amino, OMe (Me = Alkalimetall oder quaternäre Ammoniumgruppe).

Die Verbindungen (mit M = Fluor) können hergestellt werden durch Pyrolyse (bei 200 bis 600° C) der folgenden Verbindungen

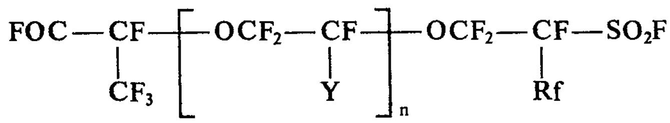

oder

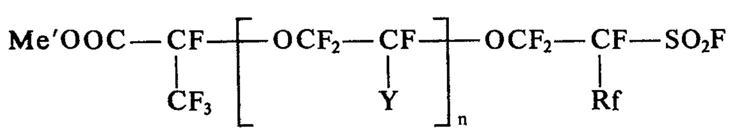

(Me' = Alkalimetall)

Bei der Pyrolyse findet folgende Reaktion statt:

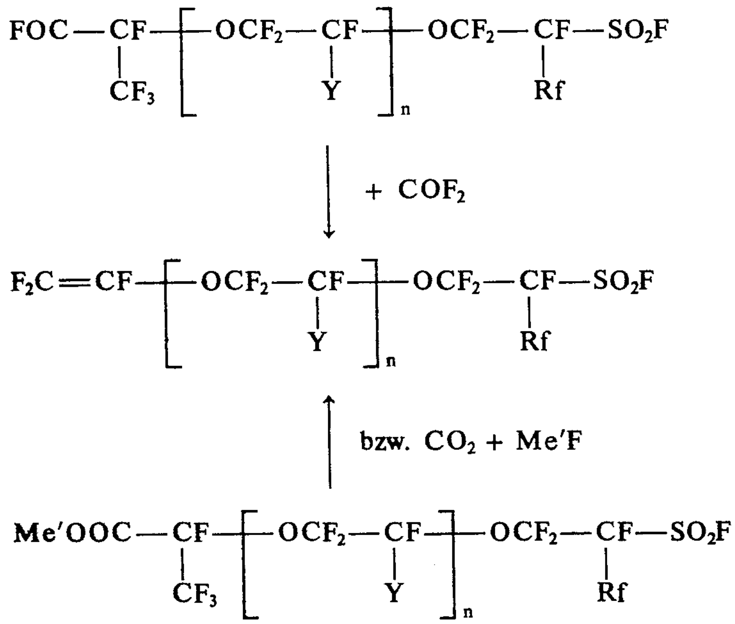


Die für die Pyrolyse verwendeten Verbindungen sind Gegenstand der US-PS 3 301 893 und werden aus perfluorierten Fluorsulfonyl-carbonsäurefluoriden und Perfluorethylenoxid (-Derivaten) hergestellt, was anhand des folgenden konkreten Beispiels illustriert sei:

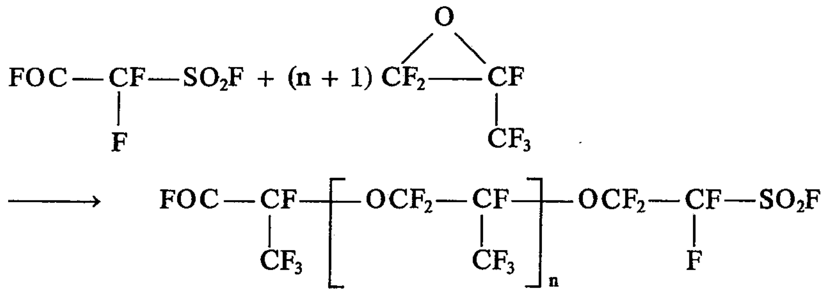

Dieses Verfahren hat aber den Nachteil, daß die als Ausgangsmaterialien verwendeten perfluorierten Fluorsulfonylcarbonsäurefluoride praktisch nur über Sultone herstellbar sind, bei deren Produktion explosive Gemische entstehen können [Chem. Eng. News 49, Heft 22, S. 3 (1971)]. Der Herstellung der perfluorierten Fluorsulfonylessigsäure beispielsweise liegt folgende Reaktion zugrunde:

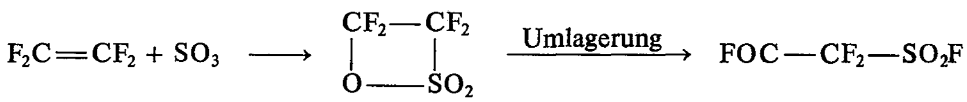

Sulton

Dabei sind Gemische aus $SO_3$ und dem Sulton instabil wegen der Reaktionsmöglichkeit:

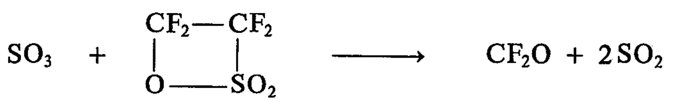

(+ Wärme!)

Weitere perfluorierte Vinylether sind aus der DE-OS 2 708 677 bekannt. Die Verbindungen besitzen die Formel

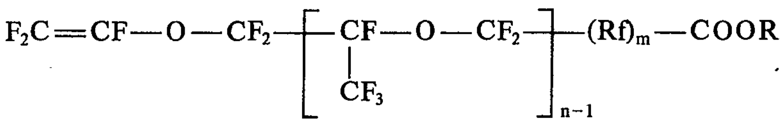

worin Rf = bifunktionelle ($C_1-C_{10}$)-Perfluorgruppe
R = Alkyl, m = 0 oder 1, n = 1–5.

Die Herstellung dieser Verbindungen erfolgt durch Pyrolyse der folgenden Verbindungen

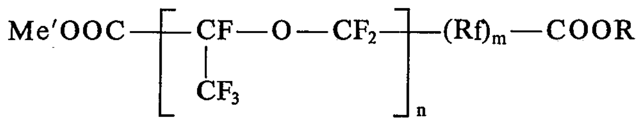

(Me′ = Alkalimetall)

die ihrerseits durch Umsetzung der entsprechenden Säurefluoride:

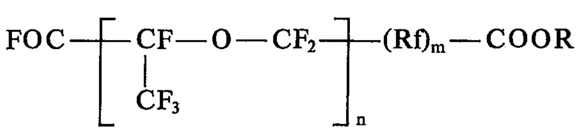

mit einem Alkalicarbonat (z. B. $Na_2CO_3$) erhalten werden.

Die Ausgangsverbindungen für diese Pyrolyse sind z. T. nach dem in der DE-OS 2 751 050 beschriebenen Verfahren erhältlich:

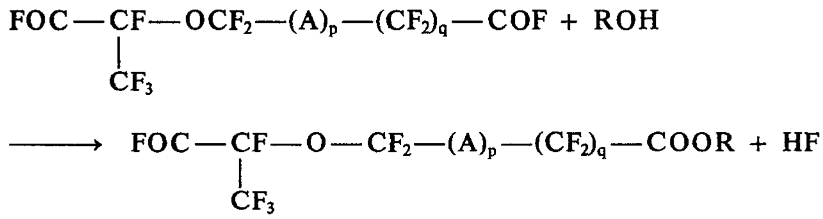

In den Formeln bedeutet: A = bifunktionelle ($C_1-C_{10}$) Perfluorgruppe, ggf. mit Etherbindungen

(z. B. die 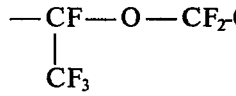-Gruppe)

R = Alkyl
p = 0 oder 1
q = 1–8.

Dieses Verfahren verläuft jedoch nicht einheitlich zu den gewünschten Verbindungen, sondern führt zu Gemischen von drei Derivaten. Die nur schwer abtrennbaren isomeren Halbester und Diester, die zusammen bis zu etwa 30% ausmachen können, sind für die weitere Synthese zu den perfluorierten Vinylethern nicht brauchbar.

Praktisch die gleichen Verbindungen sind auch offenbart in DE-OS 2 817 366, und die dort angegebene Formel ist:

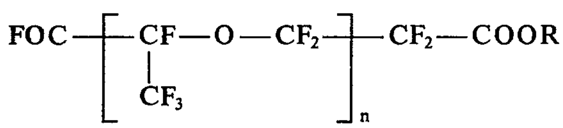

worin R = ($C_1-C_6$)-Alkyl
n = 0–6.

Die Verbindungen werden folgendermaßen hergestellt:

mit n = 0:

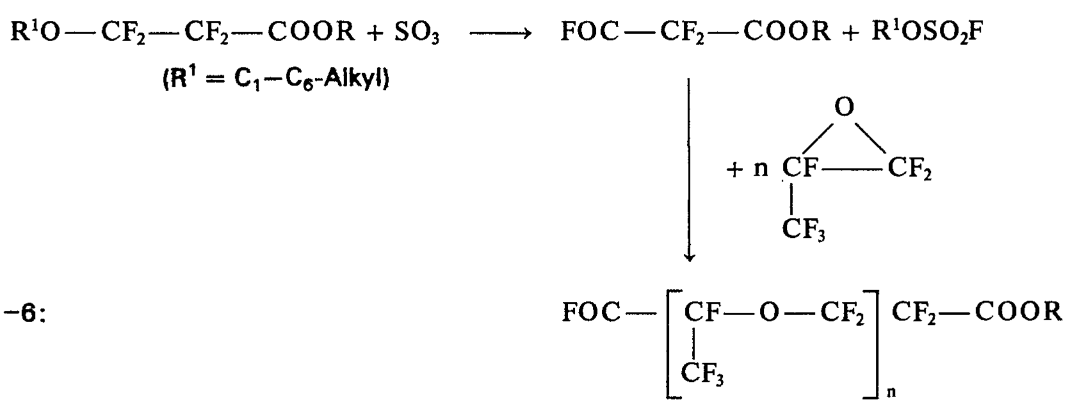


mit n = 1–6:

Praktische Bedeutung besitzt das Verfahren nur mit der Ausgangsverbindung

$CH_3O-CF_2-CF_2-COOCH_3$.

Das bei der Umsetzung mit $SO_3$ daraus in stöchiometrischer Menge entstehende Nebenprodukt $CH_3O-SO_2F$ ist eine hochtoxische Verbindung, deren Gefährlichkeit mit Dimethylsulfat und ähnlichen Methylierungsmitteln vergleichbar ist [Chem. Eng. News 56, Heft 37, S. 56 (1978)].

Außer den eingangs erwähnten perfluorierten Sulfonylfluoriden mit der Gruppierung

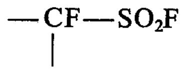

sind auch perfluorierte Fluorsulfatoverbindungen mit der Struktureinheit

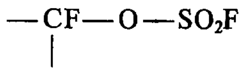

bekannt geworden. Derartige perfluorierte Fluorsulfatoverbindungen können z. B. durch anodische Oxidation von 1-H-Perfluoralkanen in einer Mischungen von Fluorsulfonsäure und einem Alkalifluorsulfonat unter Verwendung von Platinelektroden hergestellt werden (J. C. S. Chem. Comm. 1978, 118):

z. B.

$$C_5F_{11}CF_2H \xrightarrow[HSO_3F/KSO_3F]{-2e} C_5F_{11}CF_2-O-SO_2F$$

was wahrscheinlich über das intermediär gebildete Peroxydisulfuryldifluorid $FSO_2-O-O-SO_2F$ verläuft.

Derartige Fluorsulfatoverbindungen können etwa mit Alkoholen in mehreren Tagen wie folgt reagieren:

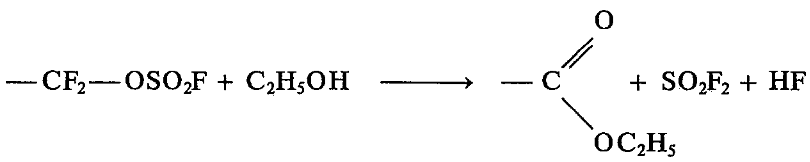

vgl. I zv. Akad. Nuak. SSSR, Ser. Khim. 1974 engl. Ausgabe S. 441/42.

Eine besondere Bedeutung haben die perfluorierten Fluorsulfatoverbindungen jedoch bisher nicht erlangt.

Wegen der erheblichen Bedeutung von perfluorierten organischen Produkten und der oft mit sicherheitstechnischen Problemen oder mit ausbeutemindernden Nebenreaktionen behafteten bekannten Verfahren, insbesondere zur Herstellung der perfluorierten Vinylether mit noch einer weiteren funktionellen Gruppe bestand die Aufgabe, einen einfacheren und verbesserten Weg zu solchen Verbindungen zu erschließen, der über keine explosiven Zwischenstufen verläuft, einheitlich nur zu den gewünschten Produkten führt und auch keine toxischen Nebenprodukte liefert.

Diese Aufgabe konnte erfindungsgemäß durch die Bereitstellung neuer bifunktioneller Perfluorverbindungen, nämlich von ω-Fluorsulfato-perfluorcarbonsäurederivaten der Formel I gelöst werden:

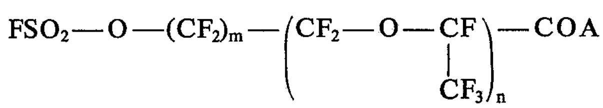

(I)

worin A = Halogen, vorzugsweise Cl oder F, insbesondere F, oder die Gruppe OR (R = Alkyl, Aryl oder Aralkyl mit vorzugsweise bis zu 10 C-Atomen, insbesondere $CH_3$ oder $C_2H_5$),

m = 1–10, vorzugsweise 1–8, insbesondere 1–6, und

n = 0–10, vorzugsweise 0–4, insbesondere 0 oder 1

bedeuten.

Für A = Halogen sind die Verbindungen ω-Fluorsulfato-perfluorcarbonsäurehalogenide,

für A = OR ω-Fluorsulfato-perfluorcarbonsäureester.

Zu entsprechenden bifunktionellen Vinylethern kommt man — ausgehend von den Estern (also mit A = OR) der Formel I — durch Zersetzung in Gegenwart eines Alkalifluorids nach dem Verfahren der gleichzeitig eingereichten Anmeldung EP-A-47 949 sowie Umsetzung mit Hexafluorpropenoxid mit nachfolgender Abspaltung von $COF_2$ auf bekannte Weise:

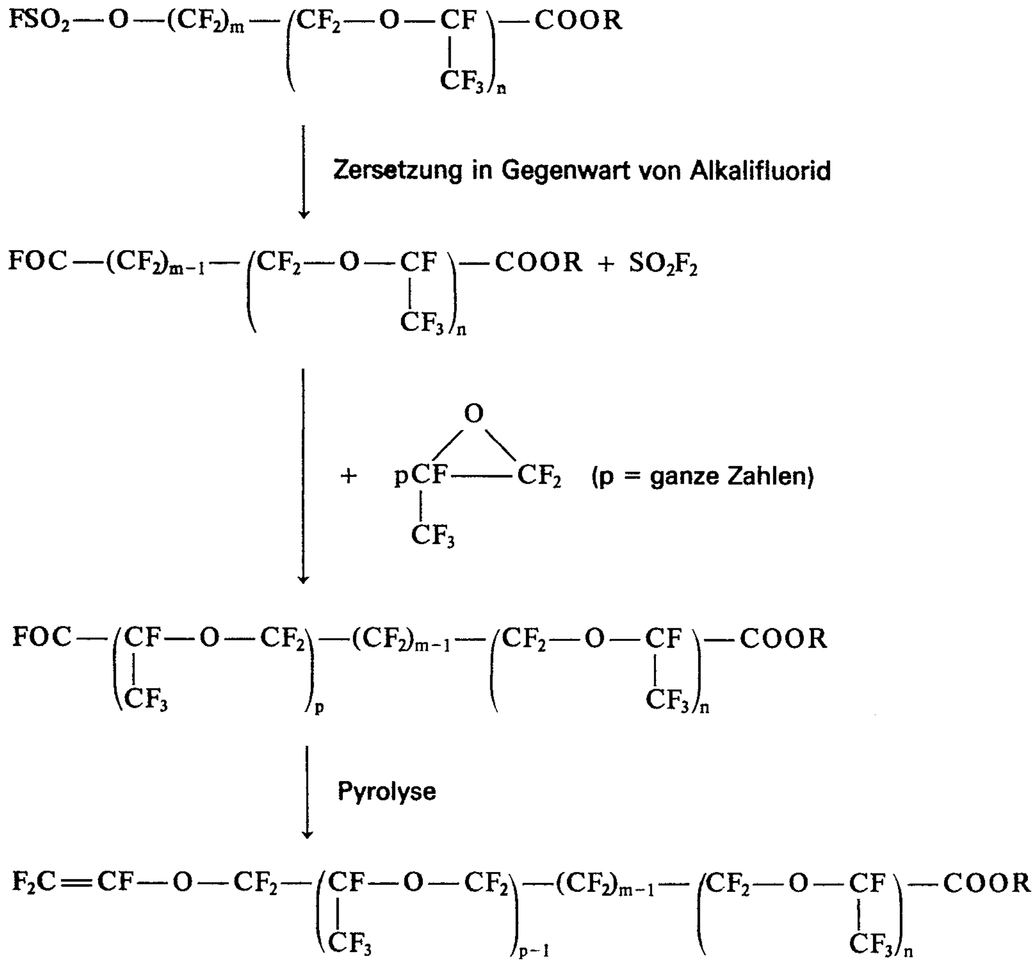


(I)

Die Verbindungen der Formel I werden erfindungsgemäß dadurch hergestellt, daß man

a) ω-H-Perfluorcarbonsäurehalogenide der Formel II

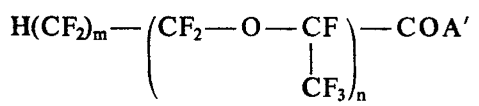

(II)

worin A' = Halogen und
m und n die gleiche Bedeutung wie in Formel I besitzen, in einem Elektrolyten aus Fluorsulfonsäure und einem Alkalifluorsulfonat unter Verwendung von Anoden aus Metallen der Platingruppe (Osmium, Iridium, Platin) und/oder glasartigem Kohlenstoff, und von Kathoden aus einem üblichen, jedoch unter den Elektrolysebedingungen stabilen Material, elektrolysiert, die dabei gebildeten ω-Fluorsulfato-perfluorcarbonsäurehalogenide der Formel III

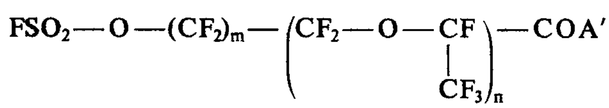

(III)

worin A' die gleiche Bedeutung wie in Formel II und m und n die gleiche Bedeutung wie in den Formeln I und II besitzen,
isoliert und
b) mit mit einer organischen Hydroxylverbindung der Formel IV

ROH (IV)

worin R die bei Formel I genannte Bedeutung besitzt, zu ω-Fluorsulfato-perfluorcarbonsäureestern der Formel I mit A = OR verestert.

Das praktisch komplikationslose Gelingen der beiden Verfahrensstufen a) und b) war

überraschend, weil

a) die Säurehalogenidfunktion der ω-H-perfluorcarbonsäurehalogenide der Formel II bei der Elektrolyse nicht angegriffen und somit nicht verändert wird und weil

b) in der Veresterungsstufe keine Reaktion der Fluorsulfatogruppe erfolgt;

letzteres war insbesondere im Hinblick auf die aus Izv. Akad. Nauk, SSSR, Ser. Khim. 1974, engl. Ausgabe, S. 441/42, bekannte Reaktion von perfluorierten ω-Fluorsulfatoverbindungen mit Alkohol zu entsprechenden Estern kaum zu erwarten.

Die Ausgangsverbindungen für das erfindungsgemäße Verfahren — also die ω-H-perfluorcarbonsäurehalogenide der Formel II — können z. B. nach folgenden bekannten Verfahrensweisen erhalten werden:

1. J. Am. Chem. Soc. 74 (1952), 1426:
   Aus Ammoniak und Tetrafluorethylen wird in Gegenwart von Kupferacetat zunächst das folgende Triazinderivat hergestellt:

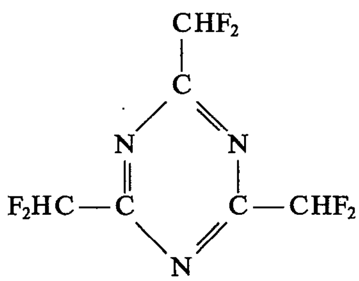

   welches dann durch Erhitzen mit wäßriger Natronlauge in das Natriumsalz der Difluoressigsäure $HCF_2-COONa$ überführt wird. Daraus sind die Säurehalogenide der Formel II (mit m = 1 und n = 0) nach bekannten Methoden erhältlich.

2. US-PS 2 559 629:
   Herstellung von aliphatischen Polyfluorcarbonsäuren und ihren Salzen durch Oxidation von Polyfluoralkanolen mit Permanganat:

$$H(CX_2CX_2)_nCH_2OH \xrightarrow{\text{Oxidation}} H(CX_2CX_2)_nCOOH$$

   X = Cl, F, mit mindestens der Hälfte der Reste X = F
   n = 1 − 3

   Die Ausgangsverbindungen für diese Oxidation werden aus Ethylenderivaten $CX_2 = CX_2$ und Methanol hergestellt.

   Aus den erhaltenen freien Säuren werden die Säurehalogenide auf bekannte Weise gewonnen; die Verbindungen mit allen Resten X = F stellen die Verbindungen der Formel II mit m = gerade Zahlen und n = 0 dar.

3. J. Org. Chem. Vol. 42 Nr. 25 (1977), 4055:
   beschreibt u. a. folgende Reaktion:

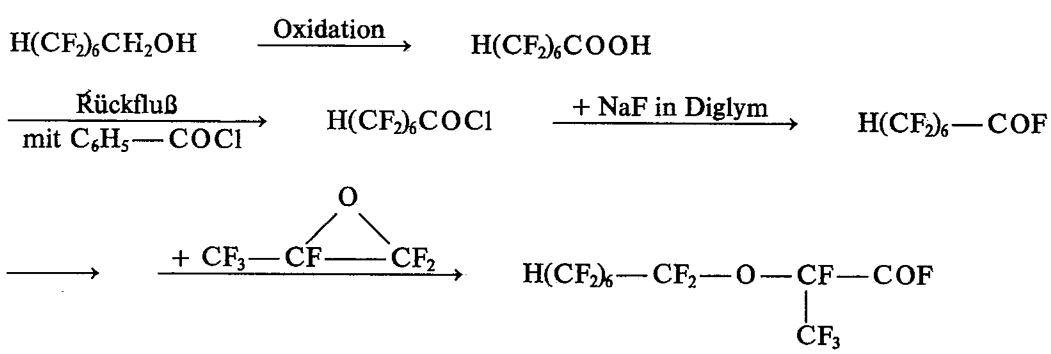


   Die letzten drei Verbindungen dieser Reaktionsreihe sind sämtlich Verbindungen der Formel II; in ganz analoger Weise sind auch die anderen unter die Formel II fallenden Verbindungen erhältlich.

Die Elektrolysestufe a) des erfindungsgemäßen Verfahrens wird im Prinzip in der etwa aus J. C. S. Chem. Comm. 1978, 118 bekannten Weise durchgeführt.

Zur Bereitung des Grundelektrolyten löst man am einfachsten ein leicht zugängliches Alkalichlorid, z. B. LiCl, NaCl oder KCl, in Fluorsulfonsäure $FSO_3H$, die man gegebenenfalls vorher einer Reinigung durch Destillation unterworfen hat, wobei der freigesetzte Chlorwasserstoff aus der Lösung entweicht bzw. durch Einleiten eines trockenen Stickstoffstroms ausgetrieben wird. Die Konzentration des Alkalisulfonats im Elektrolyten ist nicht kritisch und kann im Bereich von etwa 0,05 bis etwa 3 Mol pro Liter liegen.

Die Ausgangsstoffe der Formel II werden im Grundelektrolyten gelöst oder dispergiert, wobei bis zu etwa 60 Gew.-% II, bezogen auf den Grundelektrolyten, eingesetzt werden können.

Als Anodenmaterialien für die Elektrolyse eignen sich Platin oder Metalle der Platingruppe (Os, Ir, Pt) sowie Platinlegierungen mit bis zu etwa 90 Gew.-% an anderen Edelmetallen, insbesondere Iridium, und/oder glasartiger Kohlenstoff (»glassy carbon«). Das letztgenannte Material ist als Anodenmaterial bevorzugt, da es unter den Elektrolysebedingungen besonders korrosionsbeständig ist.

Über die Herstellung, Struktur und Eigenschaften von glasartigem Kohlenstoff siehe z. B. den Artikel »Thermischer Abbau von Polymeren bis zum elementaren Kohlenstoff — ein Weg zu Werkstoffen der Zukunft« von E. Fitzer in Angew. Chem. 92, 375 bis 386 (1980).

Das Kathodenmaterial ist für den Prozeß nicht kritisch. Deshalb können im Prinzip alle möglichen bekannten Kathodenmaterialien verwendet werden, sofern diese nur bei den hier herrschenden Elektrolysebedingungen einigermaßen stabil sind. Geeignet sind beispielsweise Platin, Kupfer, Edelstahl und glasartiger Kohlenstoff.

Das Flächenverhältnis von Anode zu Kathode liegt im allgemeinen zwischen etwa 1 : 1 und vorzugsweise etwa 5 : 1 bis etwa 10 : 1.

Ansonsten läßt sich die Elektrolyse am einfachsten in einer ungeteilten Zelle durchführen, wobei auch normale, labormäßige Becherglaszellen benutzt werden können.

Die angewandten Stromdichten liegen zweckmäßig zwischen etwa 10 und 150 mA $\times$ $cm^{-2}$, vorzugsweise zwischen etwa 20 und 80 mA $\times$ $cm^{-2}$,
die Elektrolysetemperaturen zwischen etwa 0 und 100°C, vorzugsweise zwischen etwa 20 und etwa 40°C.

Nach Beendigung der Elektrolyse wird das Reaktionsprodukt aus dem Elektrolysegemisch entweder durch Destillation oder gegebenenfalls auch durch Scheiden abgetrennt; die zurückbleibende Elektrolytphase kann nach Ergänzung mit frischer Fluorsulfonsäure für einen nachfolgenden Ansatz wiederverwendet werden.

Die so erhaltenen $\omega$-Fluorsulfato-perfluorcarbonsäurehalogenide der Formel III sind durch fraktionierte Destillation weiter reinigbar. In einigen Fällen ist die restlose Abtrennung von Fluorsulfonsäure auf destillativem Weg nur schwer möglich. Hier können dann Fluorsulfonsäure-freie $\omega$-Fluorsulfato-perfluorcarbonsäurehalogenide III etwa durch Behandeln des Rohproduktes mit NaF (für A'=F) und anschließende schonende Destillation gewonnen werden. Geringe Anteile an Fluorsulfonsäure stören jedoch im allgemeinen nicht bei der nachfolgenden Veresterungsstufe b.

Die Veresterung mit einer organischen Hydroxylverbindung — insbesondere mit Methanol oder Ethanol — wird im Prinzip auf bekannte Weise in Gegenwart oder in Abwesenheit eines inerten Lösungsmittels wie z. B. von Methylenchlorid durchgeführt. Dabei werden das $\omega$-Fluorsulfato-perfluorcarbonsäurehalogenid III und die Hydroxylverbindung IV zweckmäßig im Molverhältnis von etwa 1 : 1 bis etwa 1 : 1,5 eingesetzt. Ein höherer Überschuß der Hydroxylverbindung ist möglich, jedoch ohne besonderen Vorteil.

Für die Veresterung ist es auch praktisch ohne Belang, in welcher Reihenfolge die Reaktionskomponenten zusammengegeben werden. Bevorzugt ist jedoch, das Halogenid III in einem inerten Lösungsmittel vorzulegen und unter Kühlung mit einer Lösung der Hydroxylverbindung III in dem gleichen Lösungs- oder Verdünnungsmittel zu versetzen. Dabei soll in jedem Fall möglichst durch gutes Rühren für eine gleichmäßige Durchmischung des Ansatzes gesorgt werden.

Während der Reaktion wird die Innentemperatur des Ansatzes zweckmäßig zwischen etwa −80 und +70°C, vorzugsweise zwischen etwa −20 und +40°C, insbesondere zwischen etwa 0 und +20°C gehalten.

Da im Falle von A' = F bei der Veresterung Flußsäure entsteht, welche Borsilikatglas angreift, ist es in diesem Fall von Vorteil, die Reaktion in einem Gefäß aus flußsäurebeständigem Material durchzuführen.

Nach Beendigung der Reaktion wird die entstandene Halogenwasserstoffsäure etwa durch Waschen mit Wasser entfernt sowie die organische Phase getrocknet und destilliert.

Die Endprodukte des erfindungsgemäßen Verfahrens (Ester der Formel I mit A = OR) sind meist farblose Flüssigkeiten, die im reinen Zustand eine hohe Beständigkeit aufweisen.

Die erfindungsgemäßen neuen Verbindungen der Formel I eröffnen einen neuen, vereinfachten Weg zu an sich bekannten perfluorierten Vinylethern mit einer weiteren funktionellen Gruppe, die auf bekannte Weise zu wertvollen chemisch und thermisch beständigen Schmier- und Gleitmitteln, Überzugs- und Dichtungsmaterialien, Elastomeren, Ionenaustauschern, etc. verarbeitet werden können.

Der Vorteil des durch die erfindungsgemäßen Verbindungen I ermöglichten neuen Wegs zu an sich bekannten perfluorierten Vinylethern gegenüber den bekannten Wegen wird durch folgenden

Vergleich deutlich, wobei hier nicht der gesamte Weg, ausgehend von gemeinsamen einfachen Basisprodukten bis zu den perfluorierten Vinylethern, sondern nur bis zu den allen gemeinsamen Vinylether-Vorprodukten (perfluorierte Dicarbonsäurefluoridester) dargestellt wird.

Vergleich A: **Herstellung** von $FOC-CF_2-COOCH_3$

1. Über die erfindungsgemäße Verbindung der Formel I mit m = 2, n = 0 und A = $CH_3$

(= $FSO_2-O-CF_2-CF_2-COOCH_3$):

$CF_2{=}CF_2$

$CH_3OH$/Radikalstarter — oder: $NaCN/H_2O$

81 %, lt. US-PS 2 802 028 Ex. VI

$H-CF_2-CF_2-CH_2OH$

$KMnO_4$

$H-CF_2-CF_2-COOH$

$C_6H_5-CCl_3$ — (89%)*) nach US-PS 2 802 028 Ex. I ohne Ausbeuteangabe

$H-CF_2-CF_2-COCl$

KF — (89%)*)

– Analogieverfahren –

$H-CF_2-CF_2-COF$

Elektrolyse in $FSO_3H$/Alkali-F-Sulfonat — (74%)*)

$FSO_2-O-CF_2-CF_2-COF$ — erfindungsgemäßes Verfahren

$CH_3OH$ — (86%)*)

$FSO_2-O-CF_2-CF_2-COOCH_3$

KF — (90,4%)*) Verfahren gemäß gleichzeitig eingereichter Anm. EP-A-47 949

$FOC-CF_2-COOCH_3$

Ausbeute, bezogen auf $CF_2 = CF_2$: 36,9% d. Th.

*) In eigenen Versuchen erhaltene Ausbeuten.

2. Nach dem Stand der Technik:

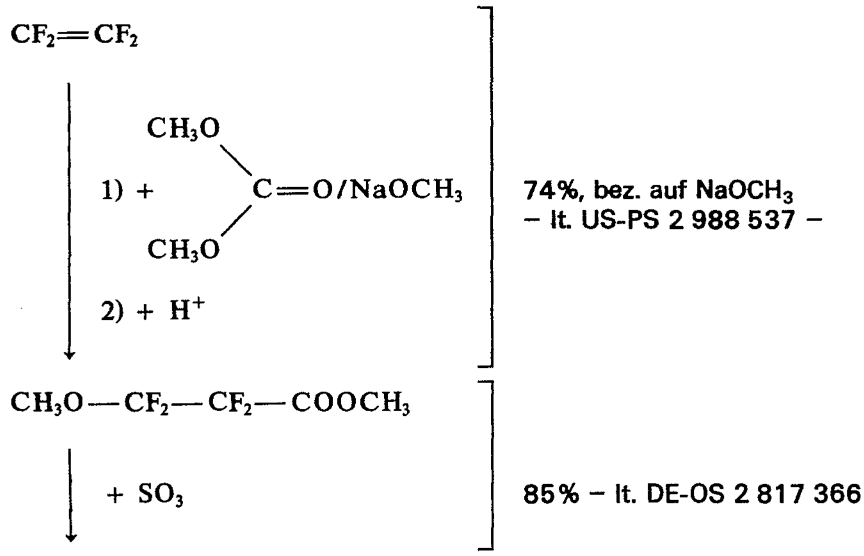


$FOC—CF_2—COOCH_3 + CH_3O—SO_2F$ (hochgiftig!)

Ausbeute an $FOC-CF_2-COOCH_3$: 62,9%.

Dabei ist folgendes zu berücksichtigen:

a) Die Menge an eingesetztem $CF_2{=}CF_2$ wurde nicht bestimmt, so daß die angegebene Ausbeute nicht auf $CF_2{=}CF_2$ bezogen werden kann. Außerdem entsteht bei dieser Reaktion ein Nebenprodukt mit mindestens 12% Ausbeute.

b) Die Ausbeute an $FOC-CF_2-COOCH_3$ in der zweiten Stufe ist gaschromatographisch aus dem Gemisch $FOC-CF_2-COOCH_3/CH_3-O-SO_2F$ bestimmt. Die Abtrennung des Fluorsulfonsäuremethylesters, der hochtoxisch ist, erfolgt durch Überleiten des Gemisches über NaF bei etwa 400°C und ist auch dann unvollständig. Für das gereinigte $FOC-CF_2-COOCH_3$ ist keine Ausbeute mehr angegeben.

Trotz dieses relativ einfach erscheinenden Wegs nach dem Stand der Technik, ist dieser jedoch insbesondere wegen der unvermeidlichen Bildung des hochtoxischen $CH_3OSO_2F$ gegenüber dem durch die Erfindung möglichen Weg ungünstiger.

Vergleich B: Herstellung von $FOC-CF_2-CF_2-CF_2-COOCH_3$

1. Über die erfindungsgemäße Verbindung der Formel I mit m = 4, n = 0 und A = $CH_3$

$(= FSO_2—O—CF_2—CF_2—CF_2—CF_2—COOCH_3)$:

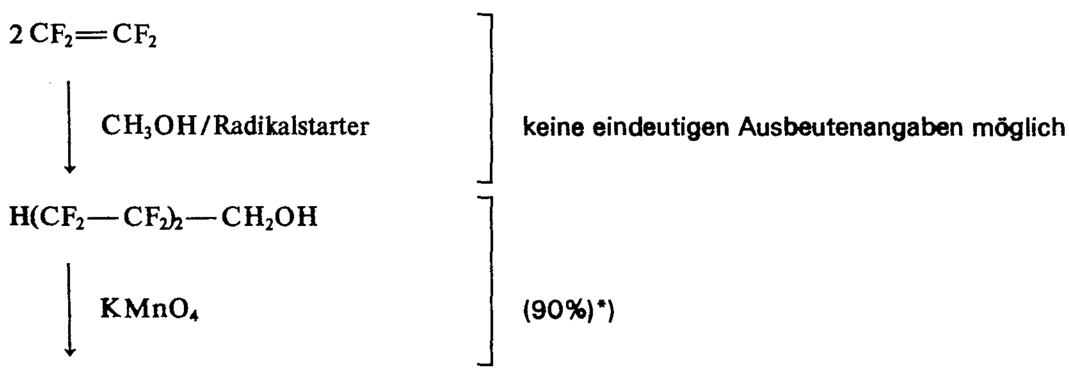

$H(CF_2—CF_2)_2—COOH$

↓ $C_6H_5—CCl_3$ — (89%)*) –lt. J. Org. Chem. 30, 2, 182 (1965) 86%

$H(CF_2—CF_2)_2—COCl$

↓ KF — (90–96%)*) – lt. J. Org. Chem. 30, 2, 182 (1965) 46%

$H(CF_2—CF_2)_2—COF$

↓ Elektrolyse in $FSO_3H$ / Alkali-F-Sulfonat — (71%)*) erfindungsgemäßes Verfahren

$FSO_2—O—(CF_2—CF_2)_2—COF$

↓ $CH_3OH$ — (86%)*)

$FSO_2—O—(CF_2—CF_2)_2—COOCH_3$

↓ KF — (88,4%)*) Verfahren gemäß Anm. EP-A-47 949

$FOC—CF_2—CF_2—CF_2—COOCH_3$

Ausbeute, bezogen auf das Alkanol $H(CF_2-CF_2)_2-CH_2OH$: 38,9%.

2. Nach dem Stand der Technik

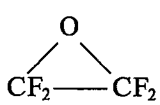

↓ LiJ — 39% lt. US-PS 3 311 658

$J—CF_2—COF$

↓ $CF_2{=}CF_2$ — 34% lt. US-PS 3 311 658

$J—CF_2—CF_2—CF_2—COF$

↓ $SO_3$ — 78% lt. DE-AS 2 642 824

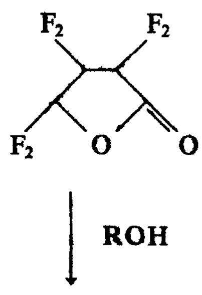


$FOC-CF_2-CF_2-COOR$

**Ausbeute ca. 6–7%.**

$R = CH_3$ bis 61%
$R = C_2H_5$ bis 71%
lt. DE-AS 2 651 531

Wegen der Kompliziertheit und der schlechten Ausbeute ist dieser Weg gegenüber dem erfindungsgemäß möglichen Weg weit unterlegen.

Das erfindungsgemäße Verfahren selbst zeichnet sich durch relativ einfache Durchführbarkeit und durchweg hohe Ausbeuten aus.

Die Erfindung stellt daher einen beträchtlichen Fortschritt auf diesem Gebiet der Technik dar.

Die folgenden Beispiele sollen nun der weiteren Erläuterung der Erfindung dienen.

## I. Herstellung von Verbindungen der Formel I mit A = Halogen

### Beispiel 1

Darstellung von Fluorsulfato-difluoracetylfluorid
($F-SO_2-O-CF_2-COF$)

Die Elektrolysevorrichtung besteht aus einem mit einem äußeren Kühlmantel und einem Deckel versehenen zylindrischen Gefäß von etwa 80 mm Innendurchmesser und 250 mm Höhe.

Auf den Deckel der Zelle ist ein als Rückflußkühler wirkender Trockeneiskühler aufgesetzt. Der Zelldeckel ist außerdem mit weiteren Schliffföffnungen zum Einführen eines Gaseinleitungsrohres, eines Thermometers und der Stromzuführungen für die Elektroden versehen. Im Abstand von 20 mm vom Boden der Zelle befindet sich die zylinderförmige Anode aus Platinnetz (Durchmesser 60 mm; Höhe 100 mm; Maschenabstand ca. 1 mm), die am Zelldeckel befestigt ist. Ein zweiter Zylinder aus Platinnetz (Durchmesser 20 mm; Höhe 100 mm) bildet die Kathode, die ebenfalls am Zelldeckel gehaltert wird. Als Rührer wird ein PTFE-umhüllter Magnetstab auf dem Zellboden benutzt. Die Kühlung der Zelle erfolgt durch einen Kühlkreislauf mit einem inerten Kühlmedium, z. B. Perchlorethylen oder Trifluortrichlorethan. Alle medienberührten Teile der Apparatur bestehen aus Glas, Platin oder PTFE. Als Grundelektrolyt dient eine Lösung von Kaliumfluorsulfonat in Fluorsulfonsäure, die folgendermaßen hergestellt wird:

In der Zelle werden 25 g (0,33 Mol) reines Kaliumchlorid vorgelegt und langsam mit 500 g destillierter Fluorsulfonsäure versetzt, wobei unter kräftigem Aufschäumen Chlorwasserstoff entweicht. Es bildet sich eine farblose Lösung, die durch Einleiten von trockenem Stickstoff von restlichem Chlorwasserstoff befreit und anschließend 3–5 Stunden bei einer Stromstärke von 8 A vorelektrolysiert wird.

In den gemäß vorstehender Beschreibung erhaltenen Grundelektrolyten leitet man bei einer Stromstärke von 8 A und einer Temperatur von 25–30° C im Verlauf von 12 Stunden 250 g (2,55 Mol) gasförmiges Difluoracetylfluorid ($CHF_2COF$) unter Rühren ein. Anschließend wird die Elektrolyse nach nochmaliger Zugabe von 250 g Fluorsulfonsäure noch 12 Stunden bei einer Stromstärke von 8 A fortgesetzt, wobei die Zellspannung von anfänglich etwa 6 Volt auf etwa 12–15 Volt ansteigt.

Bei der Rektifikation des Elektrolysegemisches über eine 0,5-m-Füllkörperkolonne erhält man 438 g Produkt, Kp 40–42° C, das aus ca. 13 Gew.-% Fluorsulfonsäure und 87 Gew.-% Fluorsulfato-difluoracetylfluorid besteht.

$^{19}F$-NMR ($CDCl_3$)* +50.04 (1F, $-O-SO_2-F$) +15.3 (1F, $-CO-F$);
−77.2 (2F, $-CF_2$);
IR (Gasspektrum): 5.25 μ (C=O); 6.70 μ (S=O).

Die Ausbeute, bezogen auf das eingesetzte Difluoracetylfluorid beträgt 77% der Theorie.

* Für alle $^{19}F$ NMR-Spektren dient $CFCl_3$ als innerer Standard.

## Beispiel 2

### Darstellung von 3-Fluorsulfato-perfluorpropansäurefluorid ($F-SO_2-O-CF_2-CF_2-COF$)

Unter Verwendung einer Elektrolysezelle und nach Bereitung eines Grundelektrolyten aus 500 g Fluorsulfonsäure und 25 g Kaliumchlorid, wie in Beispiel 1 beschrieben, werden 395 g (2,67 Mol) 3-H-Perfluorpropansäurefluorid ($H-CF_2-CF_2-COF$) 12 Stunden bei einer Stromstärke von 8 A und einer Temperatur von 20–25°C elektrolysiert. Anschließend wird noch 24 Stunden bei 5 A elektrolysiert, wobei die Zellspannung langsam von etwa 8 auf 20 Volt ansteigt. Durch kurzzeitiges Umpolen der Elektroden läßt sich die Spannung wieder auf den Ausgangswert senken. Nach beendeter Elektrolyse beträgt der Ladungsdurchgang 216 Ah. Aus dem Elektrolysegemisch werden durch Andestillieren bis zu einer Sumpftemperatur von 160°C 585 g Destillat, Kp 55–78°C erhalten. Bei der anschließenden Rektifikation über eine 0,5-m-Füllkörperkolonne werden 120 g einer Fraktion vom Kp 53–50°C erhalten, die aus 70 Gew.-% 3-Fluorsulfatoperfluorpropansäurefluorid und 30 Gew.-% Fluorsulfonsäure besteht. Die folgende Fraktion vom Kp 60–61°C enthält 420 g ca. 98%iges Fluorsulfatoperfluorpropansäurefluorid. Die Ausbeute beträgt 74% der Theorie, bezogen auf das eingesetzte Ausgangsmaterial.

$^{19}F-NMR$ ($CDCl_3$): +51.9 (1F, $-O-SO_2F$); +25.9 (1F, $-CO-F$);
−84.8 (2F, $-O-CF_2$); −119.8 (2F, $-CF_2$).
IR (Gasspektrum): 5.28 μ (C=O), 6.67 μ (S=O).

## Beispiel 3

### Darstellung von 5-Fluorsulfato-perfluorpentansäurefluorid ($F-SO_2-O-CF_2-CF_2-CF_2-CF_2-COF$)

Unter Verwendung einer Elektrolysezelle und nach Bereitung eines Grundelektrolyten aus 500 g Fluorsulfonsäure und 25 g Kaliumchlorid, wie in Beispiel 1 beschrieben, elektrolysiert man 410 g (1,65 Mol) 5–H–n-Perfluorpentansäurefluorid ($H-CF_2-CF_2-CF_2-COF$) 8 Stunden bei einer Stromstärke von 8 A, sodann nach Zusatz von 120 g Fluorsulfonsäure nochmals 12 Stunden bei 5 A.

Die Elektrolysetemperatur beträgt etwa 20–25°C. Aus dem Elektrolysegemisch werden anschließend durch Scheiden 487 g Rohprodukt als fluororganische Phase abgetrennt, dann werden aus der Elektrolytphase durch Andestillieren bis zu einer Sumpftemperatur von 160°C noch 82 g restliches Rohprodukt, Kp 56–89°C abgetrieben. Durch Rektifikation der vereinigten Rohprodukte an einer 0,5 m Füllkörperkolonne erhält man neben 65 g Ausgangsmaterial 344 g Produkt, Kp 99–101°C, das aus 95 Gew.-% 5-Fluorsulfatoperfluorpentansäurefluorid und 5 Gew.-% Fluorsulfonsäure besteht.

$^{19}F-NMR$ ($CDCl_3$): +51.5 (1F, $O-SO_2-F$); +25.8 (1F, $-COF$);
−82.9 (2F, $-O-CF_2$); −118.0 (2F, $-CF_2$); −122.3 (2F, $-CF_2$);
−124.1 (2F, $-CF_2-$).
IR (Gasspektrum): 5.3 μ (C=O); 6.65 μ (S=O).

Die Ausbeute, bezogen auf das umgesetzte 5–H–n-Perfluorpentansäurefluorid beträgt 71% der Theorie.

## Beispiel 4

### Darstellung von 5-Fluorsulfato-perfluorpentansäurechlorid ($F-SO_2-O-CF_2-CF_2-CF_2-CF_2-COCl$)

Eine Elektrolysevorrichtung, ausgestattet wie in Beispiel 1 beschrieben, besteht aus einem zylindrischen Glasgefäß von 60 mm Innendurchmesser und ca. 100 mm Höhe mit Kühlmantel, Thermometer, Gaseinleitungsrohr und Trockeneiskühler.

Anstelle eines Platinnetzes wird als Anode eine Platte (100 × 20 × 3 mm) aus glasartigem Kohlenstoff (»glassy carbon«) verwendet.

Als Kathode dient ein Stab aus glasartigem Kohlenstoff von 3 mm Durchmesser, der in einem Abstand von etwa 15 mm zur Anodenplatte angeordnet ist.

Der Grundelektrolyt besteht aus einer Lösung von Kaliumfluorsulfonat in Fluorsulfonsäure, die man durch langsame Zugabe von 250 g destillierter Fluorsulfonsäure zu 12,5 g (0,165 Mol) reinem Kaliumchlorid und Austreiben des Chlorwasserstoffs mit einem trocknen Stickstoffstrom herstellt. Anschließend wird die Lösung bei einer Stromstärke von 2 A vorelektrolysiert.

Nach Zugabe von 117 g (0,44 Mol) 5-H-Perfluorpentansäurechlorid wird bei einer Stromstärke von 2 A und einer Temperatur von 20–25°C elektrolysiert bis ein Ladungsdurchgang von 44 Ah erreicht ist. Die Zellspannung beträgt 15–19 Volt.

Nach Beendigung der Elektrolyse wird die fluororganische Phase aus dem Reaktionsgemisch durch Scheiden abgetrennt und an einer 0,5-m-Füllkörperkolonne fraktioniert destilliert. Man erhält 53 g (33% der Theorie) 5-H-Fluorsulfato-perfluorpentansäurechlorid, Kp 120—121°C.

$^{19}F-NMR$ ($CDCl_3$): +52.0 (1F, $O-SO_2-F$); −82.4 (2F, $-O-CF_2$); −112.3 (2F, $-CF_2-COCl$); −120.7 (2F, $-CF_2-$); −123.3 (2F, $-CF_2-$).

## Beispiel 5

### Darstellung von 8-Fluorsulfato-perfluor-2-methyl-3-oxaoctansäurefluorid ($F-SO_2-O-(CF_2)_5-O-CF(CF_3)-COF$)

Unter Verwendung einer Elektrolysevorrichtung und nach Bereitung eines Grundelektrolyten aus 250 g Fluorsulfonsäure und 12,5 g Kaliumchlorid, wie in Beispiel 4 beschrieben, werden 139,4 g (0,34 Mol) 8-H-Perfluor-2-methyl-3-oxa-octansäurefluorid ($H-(CF_2)_5-O-CF(CF_3)COF$) bei einer Stromstärke von 2 A, einer Zellspannung von 12—13 V und einer Temperatur von 20—25°C bis zu einem Ladungsdurchgang von 52 Ah elektrolysiert.

Aus dem Elektrolysegemisch werden durch Scheiden 182 g fluororganische Phase abgetrennt. Nach fraktionierter Destillation über eine 0,5-m-Füllkörperkolonne erhält man 138,9 g (81% der Theorie) 8-Fluorsulfato-perfluor-2-methyl-3-oxa-octansäurefluorid, Kp 147—148°C.

$^{19}-NMR$ ($CDCl_3$): +50.9 (1F, $-O-SO_2-F$); +26.6 (1F, $-CO-F$); −78.6 (1F, d. Jgem = 150 Hz); −82.5 (3F, $-CF_3$); −83.6 (2F, $-SO_2-O-CF_2-$); −86.2 (1F, d. Jgem = 150 Hz); −123.0 (2F, $-CF_2-$); −125.3 (2F, $-CF_2-$); −126.0 (2F, $-CF_2-$); −131.3 (1F, $-CF-$).

## Beispiel 6

### Darstellung von 6-Fluorsulfato-perfluor-2-methyl-3-oxahexansäurefluorid ($F-SO_2-O-(CF_2)_3-O-CF(CF_3)-COF$)

Man verwendet eine Elektrolysevorrichtung wie in Beispiel 4 beschrieben, jedoch unter Benutzung von zylindrischen Platinelektroden (Anode: Durchmesser 40 mm, Höhe 40 mm; Kathode: Durchmesser 12 mm, Höhe 40 mm).

Nach Herstellung eines Grundelektrolyten aus 250 g Fluorsulfonsäure und 12,5 g Kaliumchlorid werden 166 g (0,50 Mol) 6-H-Perfluor-2-methyl-3-oxa-hexansäurefluorid bei einer Stromstärke von 2 A, einer Zellspannung von 5—6 V und einer Temperatur von 20—25°C bis zu einem Ladungsdurchgang von 50 Ah elektrolysiert. Nach Beendigung der Elektrolyse wird die fluororganische Schicht durch Scheiden abgetrennt und an einer 0,5-m-Füllkörperkolonne fraktioniert destilliert. Man erhält 146 g (68% der Theorie) 6-Fluorsulfato-perfluor-2-methyl-3-oxahexansäurefluorid, Kp 112—114°C.

$^{19}F-NMR$ ($CDCl_3$): +50.6 (1F, $-O-SO_2-F$); +26.3 (1F, $-CO-F$); −78.2 (1F, d. Jgem = 150 Hz); −81.8 (3F, $-CF_3$); −83.4 (2F, $-SO_2-O-CF_2-$); −85.9/1F, d. Jgem. = 150 Hz); −128.1 (2F, $-CF_2-$); −130.7 (1F, $-CF-$)

## Beispiel 7

### Darstellung von 7-Fluorsulfato-perfluorheptansäurefluorid ($FSO_2-O-(CF_2)_6-COF$)

Man verwendet eine Elektrolysevorrichtung, wie in Beispiel 4 beschrieben. Der Grundelektrolyt besteht aus einer Lösung von Natriumfluorsulfonat in Fluorsulfonsäure, die man durch langsame Zugabe von 250 g Fluorsulfonsäure zu 14,6 g (0,25 Mol) Natriumchlorid und Austreiben des Chlorwasserstoffs mit einem trockenen Stickstoffstrom herstellt. Nach einer 5stündigen Vorelektrolyse bei einer Stromstärke von 2 A fügt man 43 g (0,124 Mol) 7-H-Perfluorheptansäurefluorid zu und elektrolysiert 10 Stunden bei einer Stromstärke von 2 A und einer Zellspannung von 13—16 Volt. Die Elektrolysetemperatur beträgt 20°C. Das Elektrolysegemisch wird anschließend über eine 30-cm-Füllkörperkolonne fraktioniert destilliert. Man erhält dabei 16 g einer Fraktion, die von Kp 123—158°C siedet und aus zwei Phasen besteht. Durch Redestillation dieser Fraktion wird eine ebenfalls zweiphasige Fraktion Kp 129—131°C erhalten, die in einem Scheidetrichter auf −18°C gekühlt und separiert wird. Als Unterphase erhält man 35,5 g (64% d. Th.) 7-Fluorsulfato-perfluorheptansäurefluorid.

$^{19}F-NMR$ ($CDCl_3$): +50.87 (1F, $OSO_2F$); +25.28 (1F, $-COF$); −83.41 (2F, $-CF_2-O-$); −118.41 (2F, $-CF_2-$); −121.98 (2F, $CF_2$); −122.38 (2F, $CF_2$); −122.87 (2F, $CF_2$); −124.92 (2F, $CF_2$).

## II. Herstellung von Verbindungen der Formel I mit A = OR

### Beispiel 8

Fluorsulfatodifluoressigsäuremethylester
($FSO_2-O-CF_2-COOCH_3$)

203 g eines Gemisches aus 87 Gew.-% Fluorsulfatodifluoressigsäurefluorid ( 0,9 Mol) und 17 Gew.-% Fluorsulfonsäure werden in 200 ml Methylenchlorid gelöst. Man kühlt auf +5° C ab und tropft dann eine Lösung von 49 g (1,55 Mol) Methanol in 62 ml Methylenchlorid zu. Die Innentemperatur wird durch Eiskühlung unter +10° C gehalten. Der Ansatz rührt eine Stunde bei Raumtemperatur nach. Das Reaktionsgemisch wird dreimal mit je 300 ml Wasser gewaschen und über Natriumsulfat getrocknet. Bei der Destillation über eine gute Kolonne werden 153,5 g (82%) Methylester mit Kp 119−120° C erhalten.

Analyse:

| | | | | |
|---|---|---|---|---|
| ber. | C 17,31 | H 1,45 | F 27,39 | S 15,41 |
| gef. | C 17,35 | H 1,55 | F 27,45 | S 15,65 |

| | |
|---|---|
| $^1H-NMR$ ($CDCl_3$): | 4.02 (s) |
| $^{19}F-NMR$ ($CDCL_3$): | +49.9 ($-O-SO_2F$), −76.8 ($CF_2$) |
| IR (neat): | 5.55 μ (C=O), 6.75 μ (S=O) |

### Beispiel 9

3-Fluorsulfatotetrafluorpropansäuremethylester
($FSO_2-O-CF_2-CF_2-COOCH_3$)

Zu einer auf 5° C abgekühlten Lösung von 147,6 g (0,60 Mol) 3-Fluorsulfatoperfluorpropansäurefluorid in 120 ml Methylenchlorid wird ein Gemisch aus 29 g (0,9 Mol) Methanol und 35 ml Methylenchlorid so zugetropft, daß die Innentemperatur nicht über +10° C steigt. Dabei wird mit Eis gekühlt. Der Ansatz rührt eine Stunde bei Raumtemperatur nach. Das Reaktionsgemisch wird dreimal mit je 200 ml Wasser gewaschen und über Natriumsulfat getrocknet. Die Destillation über eine gute Kolonne liefert 133 g (86%) Methylester mit einem Siedepunkt von 75−76° C (100 mm).

Analyse:

| | | | | |
|---|---|---|---|---|
| ber. | C 18,61 | H 1,17 | F 36,80 | S 12,42 |
| gef. | C 18,50 | G 1,20 | F 37,05 | S 12,65 |

| | |
|---|---|
| $^1H-NMR$ ($CDCl_3$): | 4.03 (s) |
| $^{19}F-NMR$ ($CDCl_3$): | +50.7 ($-OSO_2F$), −85 ($-O-CF_2$), −120.1 ($-CF_2-CO-$) |
| IR (neat): | 5.59 μ (C=O), 6.72 (S=O) |

### Beispiel 10

3-Fluorsulfatotetrafluorpropansäureethylester
($FSO_2-O-CF_2-CF_2-COOC_2H_5$)

Ansatz: 123 g (0,5 Mol) 3-Fluorsulfatotetrafluorpropansäurefluorid, gelöst in 100 ml Methylenchlorid
36,8 g (0,8 Mol) Ethanol, gelöst in 40 ml Methylenchlorid.

Die Reaktion wird wie in Beispiel 9 für den Methylester beschrieben durchgeführt. Es werden 109 g (77%) Ethylester mit einem Siedepunkt von 84° C (100 mm) erhalten.

Analyse:

| | | | | |
|---|---|---|---|---|
| ber. | C 22,07 | H 1,85 | F 34,90 | S 11,78 |
| gef. | C 22,00 | H 1,80 | F 34,80 | S 11,90 |

| | |
|---|---|
| $^1H-NMR$ ($CDCl_3$): | 1.04 (t, J = 7.2 Hz, 3H), 4.45 (q, J = 7,2 Hz, 2H) |
| $^{19}F-NMR$ ($CDCl_3$): | +50.8 ($-O-SO_2F$), −84.5 ($-O-CF_2$), −119.9 ($-CF_2-CO-$) |
| IR (neat): | 5.6 μ (C=O), 6.73 μ (S=O) |

## Beispiel 11

5-Fluorsulfatooctafluorpentansäuremethylester
($FSO_2-O-CF_2-CF_2-CF_2-CF_2-COOCH_3$)

Zu einer Lösung von 150 g (0,43 Mol) 5-Fluorsulfatoperfluorpentansäurefluorid in 100 ml Methylenchlorid tropft man bei 5°C eine Mischung von 26 g (0,81 Mol) Methanol und 30 ml Methylenchlorid. Es muß mit einem Eisbad gekühlt werden.

Der Ansatz rührt eine Stunde bei Raumtemperatur nach. Dann wird das Gemisch dreimal mit je 200 ml Wasser gewaschen und über Natriumsulfat getrocknet. Die Destillation über eine gute Kolonne liefert 134 g (86%) Methylester mit einem Siedepunkt von 86–87° C (50 mm).

Analyse:

| | | | | |
|---|---|---|---|---|
| ber. | C 20,12 | H 0,84 | F 47,74 | S 8,95 |
| gef. | C 20,05 | H 0,80 | F 47,35 | S 9,25 |

| | |
|---|---|
| $^1H-NMR$ ($CDCl_3$): | 3.96 (s) |
| $^{19}F-NMR$ ($CDCl_3$): | +51.5 ($-O-SO_2F$), −82.6 ($-O-CF_2$), −118 ($-CF_2-CO-$), −122.4 ($-CF_2-$), −124 ($-CF_2-$) |
| IR (neat): | 5.59 μ (C=O), 6.69 (S=O) |

## Beispiel 12

5-Fluorsulfatooctafluorpentansäuremethylester
($FSO_2-O-CF_2-CF_2-CF_2-CF_2-COOCH_3$)

Ansatz: 36 g (0,1 Mol) 5-Fluorsulfatooctafluorpentansäurechlorid, gelöst in 30 ml Methylenchlorid
4,8 g (0,15 Mol) Methanol, gelöst in 10 ml Methylenchlorid

Die Reaktion wird wie in Beispiel 10 beschrieben durchgeführt. Es werden 30 g (84%) Methylester erhalten.

## Beispiel 13

7-Fluorsulfatoperfluorheptansäuremethylester
($FSO_2-O-(CF_2)_6-COOCH_3$)

Zu einer Mischung von 40 g (0,089 Mol) 7-Fluorsulfatoperfluorheptansäurefluorid in 75 ml Methylenchlorid tropft man unter Eiskühlung eine Lösung von 7,3 g (0,23 Mol) Methanol in 30 ml Methylenchlorid. Die Innentemperatur soll +10° C nicht übersteigen. Der Ansatz rührt 2 Stunden bei Raumtemperatur nach, wird dann mehrmals mit Wasser gewaschen und über Natriumsulfat getrocknet. Die Destillation über eine Füllkörperkolonne liefert 29,6 g (72%) 7-Fluorsulfatoperfluorheptansäuremethylester mit Kp 96° C (22 mm).

Analyse:

| | | | | |
|---|---|---|---|---|
| ber. | C 20,97 | H 0,66 | F 53,91 | S 7,00 |
| gef. | C 20,7 | H 0,6 | F 54,0 | S 6,7 |

| | |
|---|---|
| $^1H-NMR$ ($CDCl_3$): | 3.98 (s) |
| $^{19}F-NMR$ ($CDCl_3$): | +50.7 (1F, $-OSO_2F$), −83.23 (2F, $-CF_2-O$), −118.72 (2F, $-CF_2-CO-$), −122.34 (4F, 2× $CF_2$), −123.0 (2F, $CF_2$), −124.9 (2F, $CF_2$) |
| IR (neat): | 5.59 μ (CO), 6.70 μ (SO) |

Beispiel 14

5-Fluorsulfatoperfluor-2-methyl-3-oxahexansäuremethylester

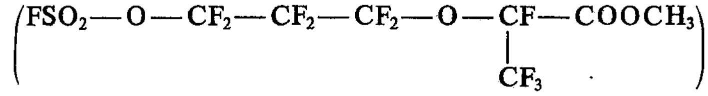

22 g (0,053 Mol) 6-Fluorsulfatoperfluor-2-methyl-3-oxahexansäurefluorid und 30 ml Methylenchlorid werden auf ca. +5° C abgekühlt. Zu dem zweiphasigen Gemisch tropft man unter Kühlung eine Lösung von 25 g (0,078 Mol) Methanol in 30 ml Methylenchlorid. Die Innentemperatur wird während der Reaktion bei 5–10°C gehalten. Der Ansatz rührt 2 Stunden bei Raumtemperatur und wird dann mehrmals mit Wasser gewaschen. Die organische Phase wird über Natriumsulfat getrocknet und destilliert. Mit Kp 158–161° C (760 torr) werden 18 g (80%) Methylester erhalten.

Analyse:

| | | | | |
|---|---|---|---|---|
| ber. | C 19,82 | H 0,71 | F 49,27 | S 7,56 |
| gef. | C 20,00 | H 0,70 | F 48,65 | S 7,75 |

$^1H-NMR$ ($CDCl_3$): 3.97 (s)
$^{19}F-NMR$ ($CDCl_3$): +51.34 ($-OSO_2F$), −78.2 (1F, $-O-CF_2-$, Jgem = 150 Hz), −81.8 ($CF_3$), −83.1 ($-CF_2-O-SO_2-$), −85.4 (1F, $-O-CF_2-$, Jgem = 150 Hz), −127.8 ($CF_2$), −131.3 (CF)
IR (neat): 5.6 μ (C=O), 6.72 μ (S=O)

Beispiel 15

8-Fluorsulfatoperfluor-2-methyl-3-oxa-octansäuremethylester

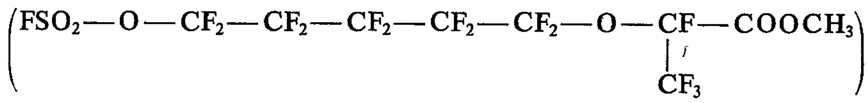

126,5 g (0,25 Mol) ω-Fluorsulfatoperfluor-2-methyl-2-oxaoctansäurefluorid und 100 ml Methylenchlorid werden auf ca. +5° C abgekühlt. Zu dem zweiphasigen Gemisch tropft man unter gutem Rühren eine Lösung von 24 g (0,75 Mol) Methanol in 30 ml Methylenchlorid. Die Innentemperatur wird durch Kühlung bei 5–10° C gehalten. Der Ansatz rührt eine Stunde bei Raumtemperatur nach und wird dann mehrmals mit Wasser gewaschen. Die organische Phase wird über Natriumsulfat getrocknet und destilliert. Mit Kp 80° C (10 torr) werden 102 g (79%) Methylester erhalten.

Analyse:

| | | | | |
|---|---|---|---|---|
| ber. | C 20,62 | H 0,58 | F 54,37 | S 6,12 |
| gef. | C 20,55 | H 0,55 | F 54,15 | S 6,30 |

$^1H-NMR$ ($CDCl_3$): 3.95 (s)
$^{19}F-NMR$ ($CDCl_3$): +51.3 ($-O-SO_2F$), −78.1 (1F, $-O-CF_2$, Jgem = 147 Hz), −82 ($CF_3$), −82.6 ($-SO_2-O-CF_2$), −85 (1F, $-O-CF_2$, Jgem = 147 Hz), −122 ($CF_2$), −124.3 ($CF_2$), −125.1 ($CF_2$), −131.3 (CF)
IR (neat): 5.58 μ (C=O), 6.7 (S=O)

**Patentansprüche**

1. ω-Fluorsulfato-perfluorcarbonsäurederivate der Formel I

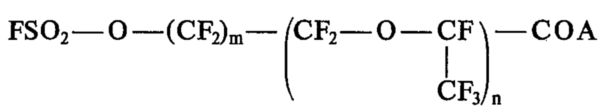

(I)

worin A = Halogen, vorzugsweise Cl oder F, insbesondere F, oder die Gruppe OR (R = Alkyl, Aryl oder Aralkyl mit vorzugsweise bis zu 10 C-Atomen, insbesondere $CH_3$ oder $C_2H_5$),
m = 1–10, vorzugsweise 1–8, insbesondere 1–6 und
n = 0–10, vorzugsweise 0–4, insbesondere 0 oder 1,

bedeuten.

2. Verfahren zur Herstellung von ω-Fluorsulfato-perfluorcarbonsäurederivaten der Formel I gemäß Anspruch 1, dadurch gekennzeichnet, daß man

a) ω-H-Perfluorcarbonsäurehalogenide der Formel II

$$H(CF_2)_m-\left(CF_2-O-\underset{\underset{CF_3}{|}}{CF}\right)_n-COA' \qquad (II)$$

worin A' = Halogen und m und n die gleiche Bedeutung wie in Formel I besitzen, in einem Elektrolyten aus Fluorsulfonsäure und einem Alkalifluorsulfonat unter Verwendung von Platin oder Metallen der Platingruppe und/oder glasartigem Kohlenstoff als Anodenmaterialien und von üblichen, jedoch unter den Elektrolysebedingungen stabilen Kathodenmaterialien elektrolysiert, die dabei gebildeten ω-Fluorsulfato-perfluorcarbonsäurehalogenide der Formel III

$$FSO_2-O-(CF_2)_m-\left(CF_2-O-\underset{\underset{CF_3}{|}}{CF}\right)_n-COA' \qquad (III)$$

worin A' die gleiche Bedeutung wie in Formel II und m und n die gleiche Bedeutung wie in den Formeln I und II besitzen, isoliert und gegebenenfalls

b) mit einer organischen Hydroxylverbindung der Formel IV

$$ROH \qquad (IV)$$

worin R die bei Formel I genannte Bedeutung besitzt, zu ω-Fluorsulfato-perfluorcarbonsäurederivaten der Formel I mit A = OR verestert.

**Claims**

1. ω-Fluorosulfato-perfluorocarboxylic acid derivatives of the formula I

$$FSO_2-O-(CF_2)_m-\left(CF_2-O-\underset{\underset{CF_3}{|}}{CF}\right)_n-COA \qquad (I)$$

in which A denotes halogen, preferably Cl or F, and in particular F, or the group OR (R = alkyl, aryl or aralkyl with preferably up to 10 C atoms, in particular $CH_3$ or $C_2H_5$), m denotes a nimber from 1 to 10, preferably from 1 to 8 and in particular from 1 to 6, and n denotes a number from 0 to 10, preferably from 0 to 4 and in particular 0 or 1.

2. A process for the preparation of ω-fluorsulfatoperfluorocarboxylic acid derivatives of the formula I as claimed in claim 1, characterized in

a) electrolyzing ω-H-perfluorocarboxylic acid halides of the formula II

$$H(CF_2)_m-\left(CF_2-O-\underset{\underset{CF_3}{|}}{CF}\right)_n-COA' \qquad (II)$$

in which A' = halogen and m and n have the same meaning as in formula I, in an electrolyte comprising fluorosulfonic acid and an alkali metal fluorosulfonate, using platinum or metals of the platinum group and/or glassy carbon as the anode materials and cathode materials which are customary, but stable under the electrolysis conditions, isolating the ω-fluorosulfato-perfluorocarboxylic acid halides thereby formed, of the formula III

$$FSO_2-O-(CF_2)_m-\left(CF_2-O-\underset{\underset{CF_3}{|}}{CF}\right)_n-COA' \qquad (III)$$

in which A' has the same meaning as in formula II and m and n have the same meaning as in the formulae I and II, and optionally

b) esterifying these compounds with an organic hydroxy compound of the formula IV

$$ROH \qquad (IV)$$

in which R has the meaning given in the case of formula I, to give $\omega$-fluorosulfato-perfluorocarboxylic acid derivatives of the formula I in which A = OR.

**Revendications**

1. Dérivés d'acides fluorosulfato-oméga perfluorocarboxyliques, caractérisés en ce qu'ils répondent à la formule I:

$$FSO_2-O-(CF_2)_m-\left(CF_2-O-\underset{\displaystyle CF_3}{\underset{|}{CF}}\right)_n-COA \qquad (I)$$

dans laquelle A représente un halogène, avantageusement Cl ou F, notamment F, ou bien le groupe OR (R étant un groupe alkyle, aryle ou aralkyle ayant avantageusement jusqu'à 10 atomes de carbone, notamment $CH_3$ou $C_2H_5$), m vaut 1 à 10, avantageusement 1 à 8, notamment 1 à 6, et n vaut 0 à 10, avantageusement 0 à 4 et en particulier 0 ou 1.

2. Procédé de préparation de dérivés d'acides fluorosulfato-oméga perfluorocarboxyliques de formule I selon la revendication 1, caractérisé en ce que:

a) on électrolyse des halogénures d'acides H-oméga perfluorocarboxyliques de formule II:

$$H(CF_2)_m-\left(CF_2-O-\underset{\displaystyle CF_3}{\underset{|}{CF}}\right)_n-COA' \qquad (II)$$

[dans laquelle A' est un halogène et m et n ont le même sens qu'à la formule I] dans un électrolyte formé à partir d'acide fluorosulfonique et d'un fluorosulfonate alcalin, en utilisant du platine ou des métaux de la famille du platine et, éventuellement ou en variante, du carbone vitreux comme matière d'anode et des matières de cathode usuelle, mais stables dans les conditions de l'électrolyse, on isole les halogénures d'acides fluorosulfato-oméga perfluorocarboxyliques de formule III ainsi formés:

$$FSO_2-O-(CF_2)_m-\left(CF_2-O-\underset{\displaystyle CF_3}{\underset{|}{CF}}\right)_n-COA' \qquad (III)$$

(dans laquelle A' a le même sens qu'à la formule II et m et n ont le même sens que dans les formules I et II), et éventuellement

b) on estérifie avec un composé hydroxylé organique de formule IV:

$$ROH \qquad (IV)$$

(dans laquelle R a le sens indiqué pour la formule I) pour obtenir des dérivés d'acides fluorosulfato-oméga perfluorocarboxyliques de formule I, dans laquelle A représente OR.